# EUROPEAN PATENT SPECIFICATION

(11) **EP 0 406 790 B1**
(45) Date of publication and mention of the grant of the patent: **13.12.1995**
(21) Application number: 90112654.0
(22) Date of filing: 03.07.1990
(51) Int. Cl.: C07D 499/88, C07D 317/40

(54) **Process for penems**
Verfahren zur Herstellung von Penemen
Procédé pour la fabrication de pénèmes

(30) Priority: 05.07.1989 GB 8915392
(43) Date of publication of application: 09.01.1991
(73) Proprietor: PHARMACIA S.p.A., 20152 Milano (IT)
(72) Inventor: Perrone, Ettore, I-20010 Boffalora Ticino (Milan) (IT); Alpegiani, Marco, I-20132 Milan (IT); Zarini, Franco, I-20019 Settimo Milanese (Milan) (IT); Mazzini, Giuseppe, I-20144 Milan (IT); Franceschi, Giovanni, I-20153 Milan (IT)
(74) Representative: Woods, Geoffrey Corlett

(56) References cited:
- EP-A- 0 199 446
- EP-A- 0 295 100
- FR-A- 2 512 027
- GB-A- 2 207 133
- CHEMICAL ABSTRACTS vol. 102, no. 25, 24 June 1985, page 593, abstract no. 220862x, Columbus, Ohio, US; & JP-A-59212488

## Description

The present invention relates to a process for preparing 2-methoxymethyl penems useful as antibacterial agents and to the preparation of the intermediates useful in the synthesis.

Prior art disclosures of the preparation of penems include GB-A-2207133, which describes a process for preparing penems which bear a pyridine substituent at the 2-position of the penem nucleus. The present invention provides a process for the preparation of compounds of formula (I): 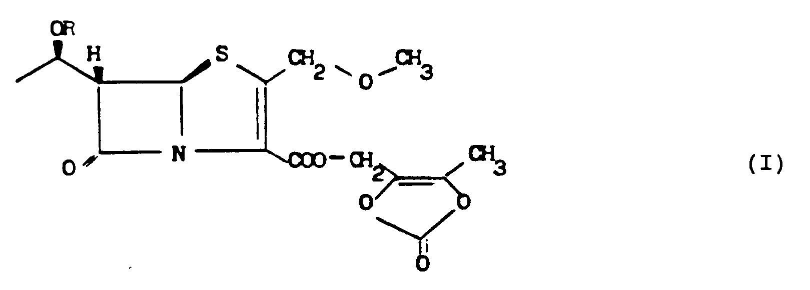
wherein R represents a hydrogen atom or a hydroxy protecting group, which process is performed as a one pot synthesis comprises reacting a compound of formula (II) 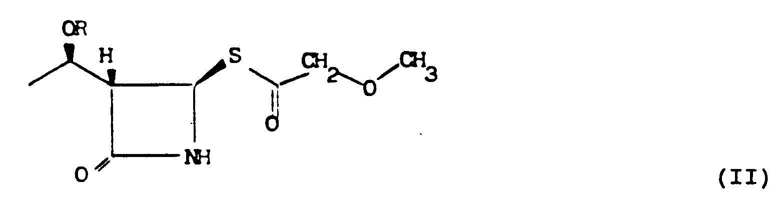
wherein R is as defined above, with an oxalyl halide in an inert solvent in the presence of an inorganic or organic base or an acid scavenger; treating the resultant compound of the formula (III) 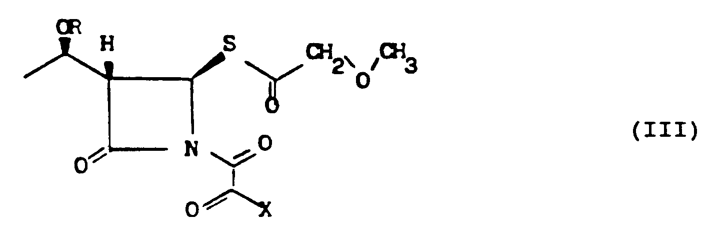
in which X is a halogen atom with a compound of the formula (IV): 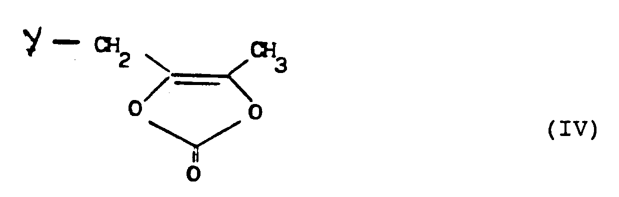
wherein Y is a hydroxy group in the presence of an acid scavenger or an organic or inorganic base; and finally cyclizing in the presence of a trialkyl phosphite the resultant compound of the formula V 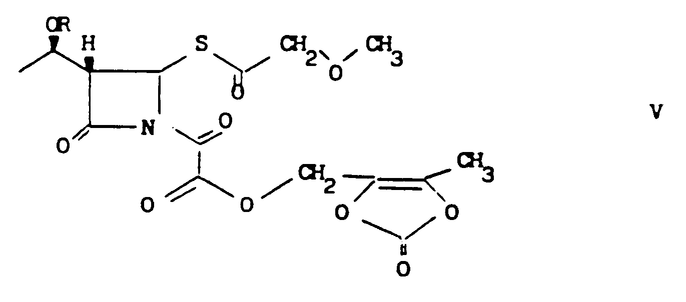
wherein R is as defined above and optionally deprotecting the resultant compound of the formula I wherein R is a hydroxy protecting group to obtain a compound of the formula I wherein R is a hydrogen atom.

The hydroxy protecting group which R may represent include:
a) a silyl group -SiR¹R²R³ wherein R¹, R², R³ are each independently C₁-C₆ alkyl, aryl, C₁-C₆ alkoxy, aryloxy or halogen atom;
b) a 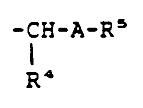 group wherein R⁴ is hydrogen, C₁-C₆ alkyl or aryl, A is oxygen or sulphur atom, R⁵ is an optionally substituted C₁-C₆ alkyl, aryl or heterocyclyl group or R⁴ and R⁵ taken together form a part of a C₅-C₆ ring
c) a 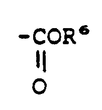 group wherein R⁶ is an optionally substituted alkyl, alkenyl, aryl, heterocyclyl or heterocyclylmethyl group
d) a 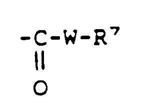 wherein R⁷ is hydrogen or as defined above under R⁶ and W is a bond, a carbonyl 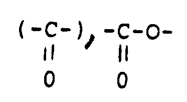 or a 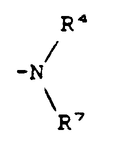 group wherein R⁴ and R⁷ are as defined above.

Preferred hydroxy protecting R groups are
a) trimethylsilyl, triethylsilyl, tert.butyldimethylsilyl, thexyldimethylsilyl, tert.butoxydiphenylsilyl
b) methoxymethyl, methylthiomethyl, benzyloxymethyl, tert.butoxymethyl, tetrahydropyranyl, tetrahyrothiopyranyl, tetrahydrofuranyl, 4-methoxytetrahydropyranyl, 1-ethoxyethyl, 1-phenoxyethyl
c) allyloxycarbonyl, benzyloxycarbonyl, trimethyl silylethoxycarbonyl, p-nitrobenzyloxycarbonyl, p-methoxybenzyloxycarbonyl, o-nitrobenzyloxycarbonyl, 3,4-dimethoxybenzyloxycarbonyl, phenoxycarbonyl, vinyloxycarbonyl, trichloroethoxycarbonyl,
d) formyl, acetyl, propionyl, butyryl, C₅-C₁₄ alkanoyl, benzoyl, phenylacetyl, phenoxyacetyl, chloroacetyl, dichloroacetyl, trichloroacetyl, trifluoroacetyl, methoxyacetyl, pivaloyl, crotonyl, acrylyl, glyoxylyl, methoxyoxalyl, allyloxyoxalyl, (5-methyl-2-oxo-1,3-dioxolen-4-yl)methoxyoxalyl, butoxyoxalyl, phenoxyoxalyl, oxamyl, carbamoyl.

In the present specification, halogen atom include bromine, chorine and iodine.

A C₁-C₆ alkyl may be methyl, ethyl, i-propyl, propyl, n-butyl, sec-butyl or ter-butyl groups.

Alkenyl may be an allyl, propenyl or butenyl group.

Aryl is a benzene ring optionally substituted by one or more methyl, nitro, methoxy, methoxycarbonyl, cyano, hydroxy, acetamido, carbamoyl.

Heterocyclyl group is 5-methyl-2-oxo-1,3-dioxolen-4-yl, furyl, thiophenyl, benzothiophenyl, thiazolyl, triazolyl, tetrazolyl, pyridyl, pyrimidyl.

The compounds of formula I and their use as antibacterial agents are described in the published European Patent Application No. 295,100.

The present invention also provides a process for preparing a compound of formula (IV) in which Y is hydroxy group, which process comprises treating a compound of the formula IV, wherein Y is a halogen atom with silver nitrate and reducing the resultant compound by treatment with zinc powder in a mixture of acetic acid and an inert organic solvent.

The process of the present invention provides penems of formula (I) in good yield and high optical purity.

It differs from the prior art in that the synthesis consists of fewer steps, even realizable in one single pot, with appreciably improved yields and industrial feasibility.

On the contrary, in the above cited EP 295,100 conversion of azetidinones II to penems I was carried out through a multistep sequence generally requiring temporary protection of the carboxylate moiety and/or proceeding in low overall yield.

The starting material of formula (II) can be conveniently prepared as described in the above cited EP-A-295,100. The present invention therefore allows the synthesis of compounds of formula (I) as summarized in the following Reaction Scheme. 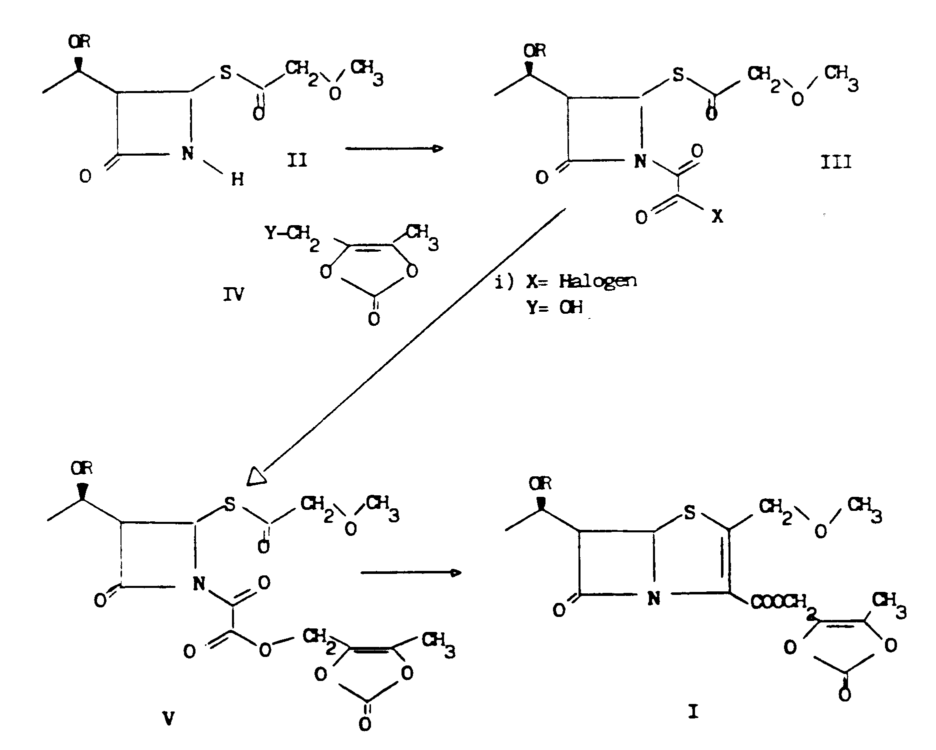
Azetidinones II are reacted in an inert solvent with oxalyl halides, especially oxalyl chloride or oxalyl bromide, in the presence of an inorganic or organic base or an acid scavenger affording halooxalyl azetidinones of formula III. Reaction of azetidinones II with oxalyl halides is performed in an aprotic solvent at a temperature ranging from -70°C to + 40°, preferably between -40°C and room temperature.

Preferred organic solvents are dichloromethane, chloroform, benzene, toluene, xylenes (as single isomer or a mixture thereof), carbon tetrachloride, ethyl acetate, propyl acetate, butyl acetate, tetrohydrofuran, dioxane, acetonitrile, diisopropylether, methylethylketone.

Said reaction is usually performed in the presence of a tertiary organic base either aliphatic or aromatic or alicyclic such as triethylamine, trimethylamine, disopropyl ethylamine, aniline, pyridine, lutidine, collidine, quinoline, N-methyl morpholine, N-methyl pyrrolidine, N-methylpiperidine, diazobicyclooctane (DABCO).

Inorganic base are also effective, preferred ones are alkaline bicarbonates or carbonates such as sodium bicarbonate, calcium carbonate, cesium carbonate, magnesium carbonate, potassium carbonate.

Amongst acid scavengers molecular sieves are the preferred ones. Mixtures of acid scavengers, inorganic bases and organic bases may be conveniently employed.

Crude solutions or mixtures of halooxalyl azetidinones III are treated with 4-hydroxymethyl-5-methyl-1,3 - dioxolen-2-one (formula IV wherein Y is hydroxy group), in the presence of an acid scavenger or an organic or inorganic base, to give the compounds of the formula V. Preferred organic or inorganic bases or acid scavengers are as described above. A preferred range of temperature is -30° to room temperature.

The resultant compound V is not generally isolated. In most cases, when not mixable with water, solutions of V are simply washed with water or aqueous solutions (brine, dilute aqueous hydrochloric acid, dilute aqueous sodium bicarbonate, etc.) to remove inorganic or ammonium halides and, if present, excess of 4-hydroxymethyl-5-methyl-1,3-dioxolen-2-one of formula IV. If mixable with water, solutions of V are concentrated in vacuo then diluted with toluene or xylene and washed with aqueous solutions.

The compound of formula V is cyclised in the presence of a trialkylphosphite such as a tri(C₁-C₄ alkyl) phosphite. Prior to treatment with a trialkylphosphite, solutions of V are generally dried over a dehydrating agent such as sodium sulphate, magnesium sulphate or calcium sulphate or by azeotropic removal.

Eventually solutions of V, following additions of trialkylphosphites, preferably trimethylphosphite or triethylphosphite, are heated at a temperature ranging from +60°to +150°C, providing penems I which are isolated by crystallization or silica gel chromatography in high overall yield.

The optional final removal of the protecting group R may be carried out by known methods, such as, for instance hydrogenolysis, e.g. in the presence of palladium on charcoal as catalysts or by hydrolysis, either acid hydrolysis, e.g. with acetic acid or oxalic acid, or neutral hydrolysis in the presence of SiO₂, or basic hydrolysis, or hydrolysis under reductive conditions, for example by the use of Fe/NH₄Cl, Zn/H⁺ or of Na₂S₂O₄, or by desilylation with fluoride salts, such as KF or (But)₄NF.3H₂O.

All the above cited reactions are preferably carried out in an inert atmosphere e.g. under nitrogen or argon and in the absence of moisture. The resulting penem of formula I, useful as an antibacterial agent, may be formulated as a pharmaceutical composition. The pharmaceutical composition also comprises a pharmaceutically acceptable carrier or diluent.

The compound of formula IV in which Y is a hydroxy group may be also obtained as described in JP-A-59-212488 (1984). The starting compounds of the formula IV in which Y is a halogen atom or a leaving group are described in Chem. Pharm. Bull 32, 2241 (1984), 36 394 (1988) and Chemical Abstracts 101:110894f.

The following Examples illustrate the invention.

### Example 1

### (5-methyl-2-oxo-1,3-dioxolen-4-yl)methyl(5R,6S)-6-[(1R)tert-butyldimethylsilyloxyethyl]-2-methoxymethylpenem-3-carboxylate.

To a stirred solution of (3S,4R)-3-[(1R)tert-butyl dimethylsilyloxyethyl]-4-(methoxyacetyl)thioazetidin-2-one (8.2g) in dry toluene (150 ml) at 5°C under nitrogen, oxalyl chloride (2.1 ml) was added, soon followed by the dropwise addition of triethylamine (6.6 ml) in toluene (30 ml). After 20 minutes a solution of 4-hydroxymethyl-5-methyl-1,3-dioxolen-2-one (6g) in methylene chloride (20 ml) was added and the resulting mixture was stirred 20 minutes at 30°C.

The organic solution was washed with water (250 ml) then dried over sodium sulphate, and concentrated to 1/3 of the initial volume.

Triethylphosphite (7 ml) in xylene (80 ml) was added and the solution was heated at reflux for 2 hours. Cyclohexane (100 ml) was added to the cooled reaction mixture. After washing with water (2x300 ml), the organic phase was concentrated and chromatographed over silica gel (eluting with cyclohexane-ethyl acetate mixtures) affording the title product as an oil (6.7 g).

### Example 2

### (5-methyl-1,3-dioxolen-2-on-4-yl) methyl (5R,6S)-6-[(1R) hydroxyethyl]-2-methoxymethylpenem-3-carboxylate.

A solution of (5-methyl-2-oxo-1,3- dioxolen-4-yl)methyl (5R,6S)-[(1R)tert-butyldimethylsilyloxyethyl]-2-methoxymethylpenem-3-carboxylate (6g) in tetrahydrofuran (30 ml) was treated with acetic acid (1 ml) and tetrabutylammonium fluoride trihydrate (3g) and left to stand at room temperature (r.t.) for 40 h. The reaction mixture was poured into water-ethyl acetate.

The organic phase was dried, concentrated and chromatographed (SiO₂, cyclohexane - ethyl acetate mixtures as eluent) to afford the title product as white powder (3.5 g)
[α]_{D} = +139° 1% MeOH, 23°C)

### Example 3

### 4-hydroxymethyl-5-methyl-1,3-dioxolen-2-one

A solution of 4-bromomethyl-5-methyl-1,3-dioxolen-2-one (960 mg) in dry CH₃CN (15 ml) was treated with silver nitrate (830 mg). The mixture was stirred 15 minutes at r.t. diluted with benzene (20 ml) and filtered.

The filtrate was heated at reflux for 2.5 h, then cooled to 20°C and treated with acetic acid (10 ml) and excess zinc powder.

Stirring was continued for few hours at r.t. (until completion of the reaction: TLC monitoring: ethyl acetate - cyclohexane 1:1, detection with agueous KMnO₄).

Removal of the solvent in vacuo and flash chromatography of the residue afforded a light yellow oil (470 mg).

Alternatively the mixture was filtered, concentrated, and the residue purified by distillation under reduced pressure (108°C / 0.5 mmHg)
IR (CHCl₃)ν ₘₐₓ 3300-3600, 1810 (strong), 1740 cm⁻¹
- NMR (CDCl₃, 90 MHz): δ : 2.13 (3H, s) 2.6 (1H, br.s, exch.D₂O) 4.30 (2H, S)

## Claims

1. A process for the preparation of a compound of formula (I): 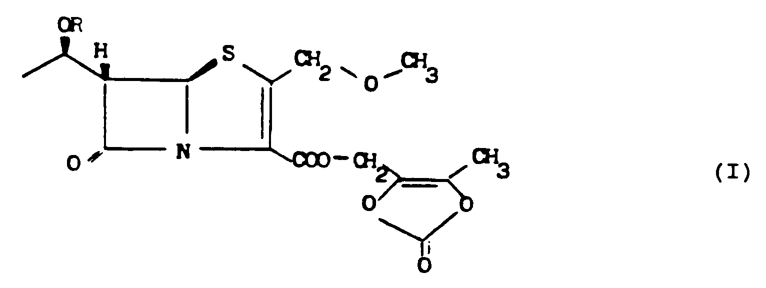 wherein R represents a hydrogen atom or a hydroxy protecting group, characterised in that the process is performed as a one pot synthesis comprises reacting a compound of formula (II): 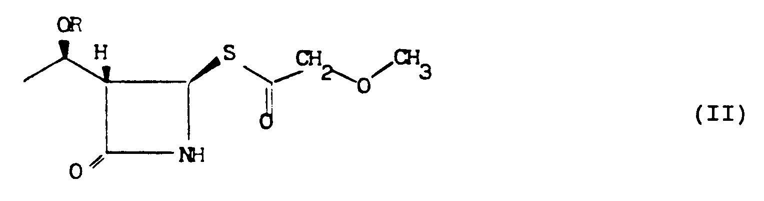 wherein R is as defined above, with an oxalyl halide in an inert solvent in the presence of an inorganic or organic base or an acid scavenger; treating the resultant compound of the formula (III): 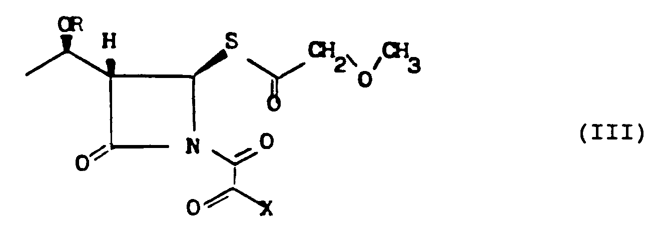 in which X is a halogen atom with a compound of the formula (IV): 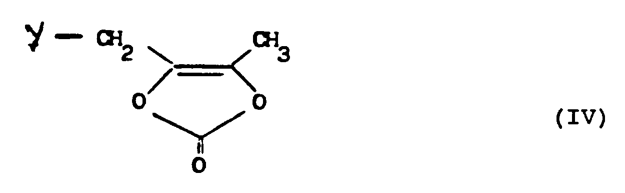 wherein Y is a hydroxy group in the presence of an acid scavenger or an organic or inorganic base; cyclizing, in the presence of a trialkylphosphite, the resultant compound of the formula (V): 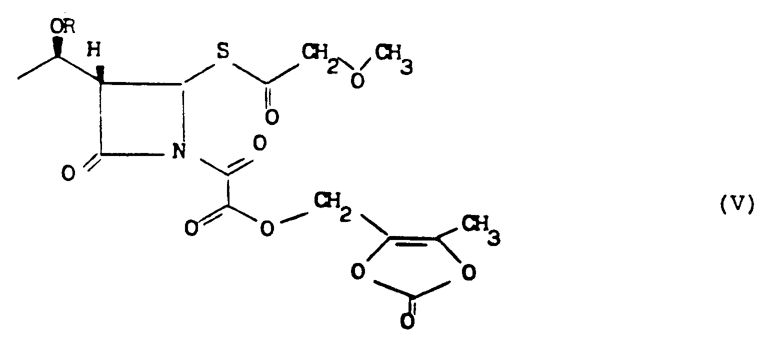 wherein R is as defined above and optionally deprotecting the resultant compound of the formula (I) wherein R is a hydroxy protecting group to obtain a compound of the formula (I) wherein R is a hydrogen atom.

2. A process according to claim 1 in which the oxalyl halide is oxalyl chloride or oxalyl bromide.

3. A process according to claim 1 or 2 in which the compound of formula (III) is reacted with the compound of formula (IV) in the presence of molecular sieves as an acid scavenger and/or an organic and/or inorganic base selected from tertiary organic bases and alkaline bicarbonates or carbonates.

4. A process for the preparation of a compound of formula (IV) as defined in claim 1 in which Y is a hydroxy group, characterised in that the process comprises treating a compound of formula (IV) as defined in claim 1 in which Y is a halogen atom with silver nitrate and reducing the resultant compound by treatment with a zinc powder in a mixture of acetic acid and an inert organic solvent.

## Patentansprüche

1. Verfahren zur Herstellung einer Verbindung der Formel (I): 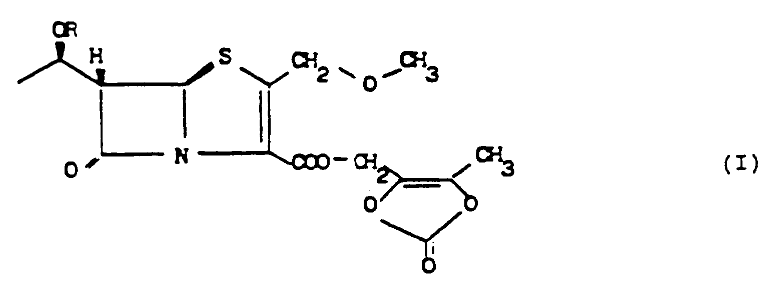 worin R ein Wasserstoffatom oder eine Hydroxy-Schutzgruppe darstellt,
dadurch **gekennzeichnet**, daß
das Verfahren als 1-Topf-Synthese durchgeführt wird, wobei man eine Verbindung der Formel (II): 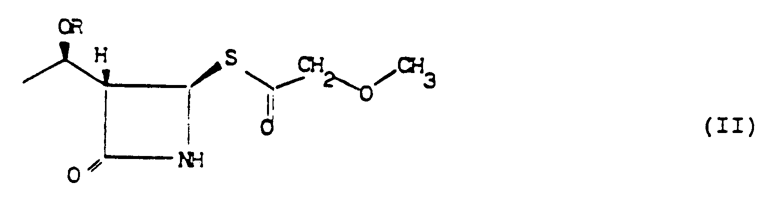 worin R wie oben definiert ist, mit einem Oxalylhalogenid in einem inerten Lösungsmittel in der Gegenwart einer anorganischen oder organischen Base oder eines Säure-Abfangmittels zur Reaktion bringt, die entstandene Verbindung der Formel (III): 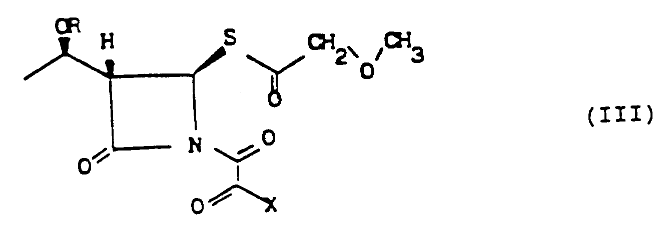 worin X ein Halogenatom ist, mit einer Verbindung der Formel (IV): 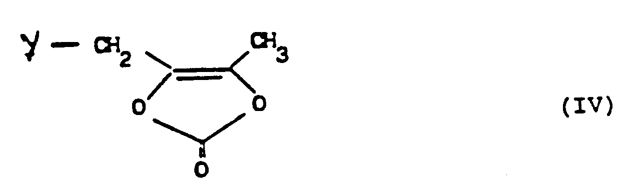 worin Y eine Hydroxygruppe ist, in der Gegenwart eines Säure-Abfangmittels oder einer organischen oder anorganischen Base behandelt und die entstandene Verbindung der Formel (V) in der Gegenwart eines Trialkylphosphits zyklisiert: 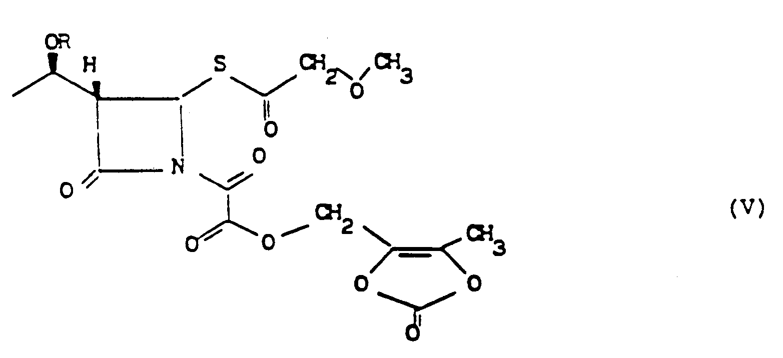 worin R wie oben definiert ist, und man die entstandene Verbindung der Formel (I), worin R eine Hydroxy-Schutzgruppe ist, gegebenenfalls einer Abspaltungsreaktion der Schutzgruppe unterzieht, um eine Verbindung der Formel (I) zu erhalten, worin R ein Wasserstoffatom ist.

2. Verfahren gemäß Anspruch 1, worin das Oxalylhalogenid Oxalylchlorid oder Oxalylbromid ist.

3. Verfahren gemäß Anspruch 1 oder 2, worin die Verbindung der Formel (III) mit der Verbindung der Formel (IV) in der Gegenwart von Molekularsieben als Säure-Abfangmittel und/oder einer organischen und/oder anorganischen Base, die aus tertiären organischen Basen und Alkalibicarbonaten oder - carbonaten ausgewählt sind, zur Reaktion bringt.

4. Verfahren zur Herstellung einer in Anspruch 1 definierten Verbindung der Formel (IV), worin Y eine Hydroxygruppe ist,
dadurch **gekennzeichnet**, daß
das Verfahren eine Stufe umfaßt, wobei man eine Verbindung der in Anspruch 1 definierten Formel (IV), worin Y ein Halogenatom ist, mit Silbernitrat behandelt und die entstandene Verbindung durch Behandlung mit Zinkpulver in einer Mischung aus Essigsäure und einem inerten organischen Lösungsmittel reduziert.

## Revendications

1. Procédé de préparation d'un composé répondant à la formule (I) : 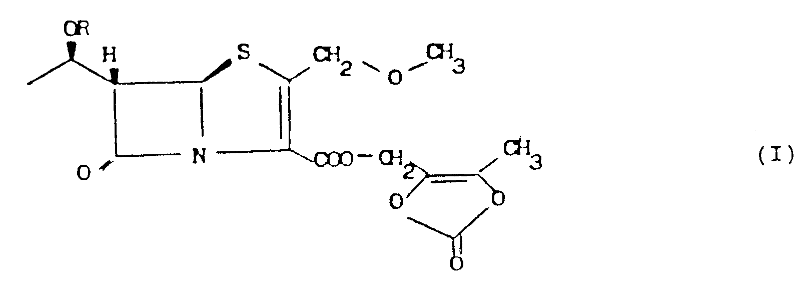 dans laquelle R représente un atome d'hydrogène ou un groupe protecteur du groupe hydroxy, caractérisé en ce que le procédé est effectué sous la forme d'une synthèse en un seul récipient, comprenant la réaction d'un composé répondant à la formule (II) : 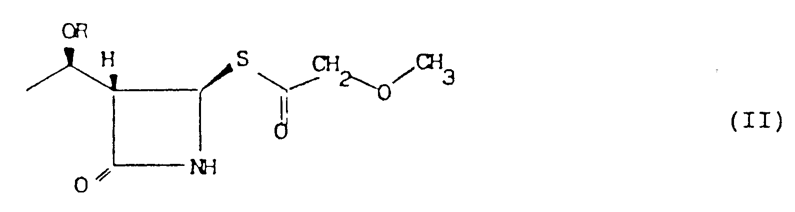 dans laquelle R est tel que défini ci-dessus, avec un halogénure d'oxalyle dans un solvant inerte, en présence d'une base minérale ou organique ou d'un fixateur d'acide; le traitement du composé obtenu, répondant à la formule (III) : 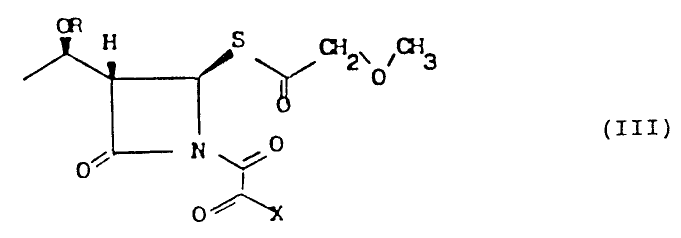 dans laquelle X est un atome d'halogène, par un composé répondant à la formule (IV) : 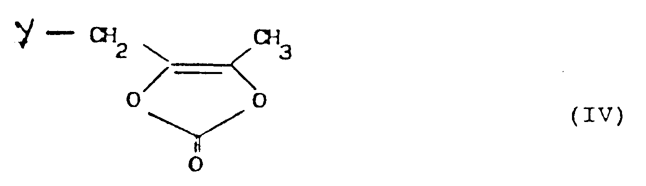 dans laquelle Y est un groupe hydroxy, en présence d'un fixateur d'acide ou d'une base organique ou minérale; la cyclisation, en présence d'un trialkylphosphite du composé obtenu répondant à la formule (V) : 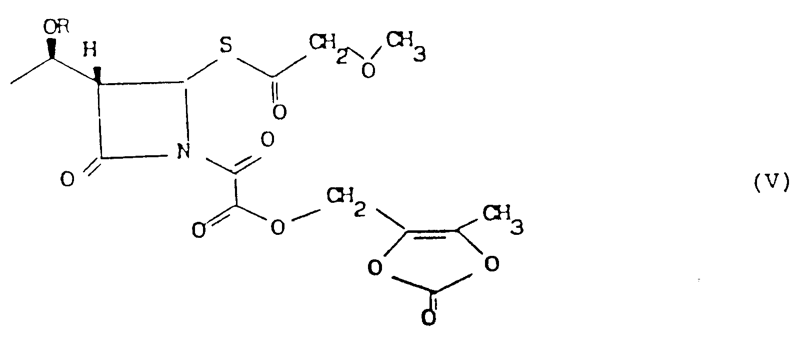 dans laquelle R est tel que défini ci-dessus ; et éventuellement la déprotection du composé obtenu de formule (I), dans laquelle R est un groupe protecteur du groupe hydroxy, pour obtenir un composé répondant à la formule (I) dans laquelle R est un atome d'hydrogène.

2. Procédé selon la revendication 1, dans lequel l'halogénure d'oxalyle est le chlorure d'oxalyle ou le bromure d'oxalyle.

3. Procédé selon la revendication 1 ou 2, dans lequel le composé répondant à la formule (III) est mis à réagir avec le composé répondant à la formule (IV), en présence de tamis moléculaire comme fixateur d'acide, et/ou d'une base organique et/ou minérale choisie parmi des bases organiques tertiaires et des bicarbonates ou carbonates alcalins.

4. Procédé de préparation d'un composé répondant à la formule (IV) telle que définie dans la revendication 1, dans laquelle Y est un groupe hydroxy, caractérisé en ce qu'il comprend le traitement d'un composé répondant à la formule (IV) telle que définie dans la revendication 1, dans laquelle Y est un atome d'halogène, par du nitrate d'argent, et la réduction du composé obtenu par traitement par de la poudre de zinc dans un mélange d'acide acétique et d'un solvant organique inerte.
